# EUROPEAN PATENT SPECIFICATION

(11) **EP 4 045 497 B1**
(45) Date of publication and mention of the grant of the patent: **22.11.2023**
(21) Application number: 20796515.3
(22) Date of filing: 15.10.2020
(51) Int. Cl.: C07D 403/12, C07D 413/12, A01N 43/54

(54) **HERBICIDAL COMPOUNDS**
HERBIZIDE VERBINDUNGEN
COMPOSÉS HERBICIDES

(30) Priority: 17.10.2019 GB 201915043
(43) Date of publication of application: 24.08.2022
(73) Proprietor: Syngenta Crop Protection AG, 4058 Basel (CH)
(72) Inventor: MORRIS, James, Alan, Bracknell Berkshire RG42 6EY (GB); BLACK, Janice, Bracknell Berkshire RG42 6EY (GB); RUSSELL, Sally, Elizabeth, Bracknell Berkshire RG42 6EY (GB); WHALLEY, Louisa, Bracknell Berkshire RG42 6EY (GB); NG, Sean, Bracknell Berkshire RG42 6EY (GB)
(74) Representative: SYNGENTA IP
(86) International application number: PCT/EP2020/079101
(87) International publication number: WO 2021/074325

(56) References cited:
- WO-A1-94/17059
- WO-A1-2015/108779

## Description

The present invention relates to novel herbicidal compounds, to processes for their preparation, to herbicidal compositions which comprise the novel compounds, and to their use for controlling weeds, in particular in crops of useful plants, or for inhibiting plant growth.

Herbicidal heteroaryl-substituted phenoxypyrimidines are disclosed in, for example, WO94/17059, WO2015/089003 and WO2015/108779. The present invention relates to novel herbicidal phenoxypyrimidine isomers which show improved properties compared to the known phenoxypyrimidine compounds.

Thus, according to the present invention there is provided a compound of Formula (I): 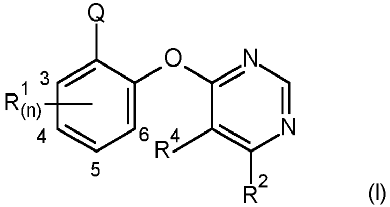 or an agronomically acceptable salt thereof,
wherein
Q is a 5-membered aromatic heterocyclic ring selected from the group consisting of: 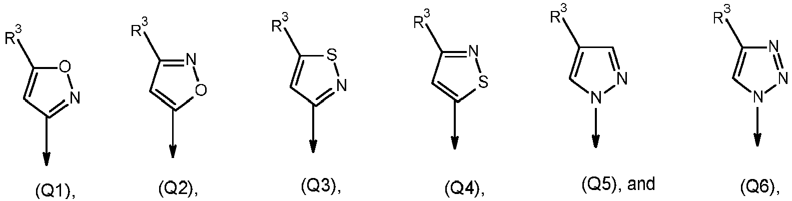
each R¹ is independently selected from the group consisting of halogen, -CN, nitro, C₁-C₄alkyl, C₂-C₄alkenyl, C₂-C₄alkynyl, C₁-C₄haloalkyl, C₁-C₄alkoxy-, C₁-C₄haloalkoxy- and -S(O)ₚC₁-C₄alkyl;
R² is selected from the group consisting of hydrogen, halogen, C₁-C₄alkyl, -NR⁵R⁶, C₁-C₄haloalkyl and C₁-C₄alkoxy;
R³ is selected from the group consisting of hydrogen, C₁-C₄alkyl, C₂-C₄alkenyl, C₂-C₄alkynyl, cyclopropyl, C₁-C₂haloalkyl, C₁-C₂alkoxy-, C₁-C₂haloalkoxy-, halogen, - C(O)C₁-C₄alkyl, NO₂, -CH₂CN, -CN and -S(O)ₚC₁-C₄alkyl;
R⁴ is selected from the group consisting of hydrogen, halogen, -OCF₃, -OCF₂H and - CN;
R⁵ is hydrogen or C₁-C₄alkyl;
R⁶ is hydrogen or C₁-C₄alkyl;
n = 0, 1 or 2; and
p = 0, 1 or 2.

C₁-C₄alkyl- includes, for example, methyl (Me, CH₃), ethyl (Et, C₂H₅), n-propyl (n-Pr), isopropyl (i-Pr), n-butyl (n-Bu), isobutyl (i-Bu), sec-butyl and tert-butyl (t-Bu). C₁-C₂alkyl is methyl (Me, CH₃) or ethyl (Et, C₂H₅).

Halogen (or halo) includes, for example, fluorine, chlorine, bromine or iodine. The same correspondingly applies to halogen in the context of other definitions, such as haloalkyl.

C₁-C₄haloalkyl- includes, for example, fluoromethyl, difluoromethyl, trifluoromethyl, chloromethyl, dichloromethyl, trichloromethyl, 2,2,2-trifluoroethyl, 2-fluoroethyl, 2-chloroethyl, pentafluoroethyl, 1,1-difluoro-2,2,2-trichloroethyl, 2,2,3,3-tetrafluoropropyl and 2,2,2-trichloroethyl, heptafluoro-n-propyl and perfluoro-n-hexyl. C₁-C₂haloalkyl is, for example, fluoromethyl, difluoromethyl, trifluoromethyl, chloromethyl, dichloromethyl, trichloromethyl, 2,2,2-trifluoroethyl, 2-fluoroethyl, 2-chloroethyl, pentafluoroethyl, or 1,1-difluoro-2,2,2-trichloroethyl.

C₁-C₂alkoxy is methoxy or ethoxy.

C₁-C₂haloalkoxy- includes, for example, fluoromethoxy, difluoromethoxy, trifluoromethoxy, 2,2,2-trifluoroethoxy, 1,1,2,2-tetrafluoroethoxy, 2-fluoroethoxy, 2-chloroethoxy, 2,2-difluoroethoxy or 2,2,2-trichloroethoxy, preferably difluoromethoxy, 2-chloroethoxy or trifluoromethoxy.

C₃-C₆cycloalkyl- includes cyclopropyl (*c*-propyl (*c*-Pr)), cyclobutyl (*c*-butyl (*c-*Bu)), cyclopentyl (*c*-pentyl) and cyclohexyl (*c*-hexyl).

C₂-C₄alkenyl- includes, for example, -CH=CH₂ (vinyl) and -CH₂-CH=CH₂ (allyl).

C₂-C₄alkynyl- includes, for example, -C=CH (ethynyl) and -CH₂-C≡CH (propargyl).

C₁-C₄alkyl-S- (alkylthio) includes, for example, methylthio, ethylthio, propylthio, isopropylthio, n-butylthio, isobutylthio, sec-butylthio or tert-butylthio, preferably methylthio or ethylthio.

C₁-C₄alkyl-S(O)- (alkylsulfinyl) includes, for example, methylsulfinyl, ethylsulfinyl, propylsulfinyl, isopropylsulfinyl, n-butylsulfinyl, isobutylsulfinyl, sec-butylsulfinyl or tert-butylsulfinyl, preferably methylsulfinyl or ethylsulfinyl.

C₁-C₄alkyl-S(O)₂- (alkylsulfonyl) includes, for example, methylsulfonyl, ethylsulfonyl, propylsulfonyl, isopropylsulfonyl, n-butylsulfonyl, isobutylsulfonyl, sec-butylsulfonyl or tert-butylsulfonyl, preferably methylsulfonyl or ethylsulfonyl.

In a more preferred embodiment of the present invention there is provided a compound of Formula (I), wherein Q is selected from the group consisting of Q1, Q2 and Q5, more preferably Q2.

In a preferred embodiment R³ is C₁-C₂haloalkyl. In a more preferred embodiment R³ is difluoromethyl or trifluoromethyl.

In one embodiment of the present invention, n is 1 and R¹ is selected from the group consisting of fluoro, chloro, bromo and CN. In a more preferred embodiment n is 1 and the R¹ substituent is present in the 3-position. In an even more preferred embodiment R¹ is 3-CN or 3-fluoro.

In one embodiment of the present invention, R² is methyl or methoxy.

In one embodiment of the present invention, R⁴ is selected from the group consisting of hydrogen, fluorine and chlorine.

Thus, in a preferred embodiment of the present invention the compound of Formula (I) is a compound of Formula (Ia): 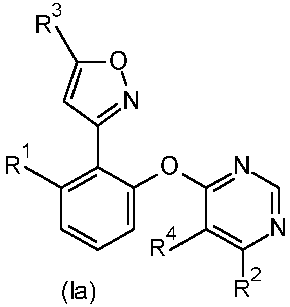 wherein R¹ is selected from the group consisting of hydrogen, fluoro, chloro and bromo, R² is methyl or methoxy, R³ is CF₃ or CF₂H and R⁴ is hydrogen or -CN, preferably hydrogen.

In another preferred embodiment of the present invention the compound of Formula (I) is a compound of Formula (Ib): 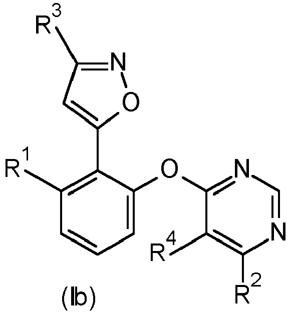 wherein R¹ is selected from the group consisting of hydrogen, fluoro, chloro and bromo, R² is methyl or methoxy, R³ is CF₃ or CF₂H and R⁴ is hydrogen or -CN, preferably hydrogen.

In another preferred embodiment of the present invention the compound of Formula (I) is a compound of Formula (Ic): 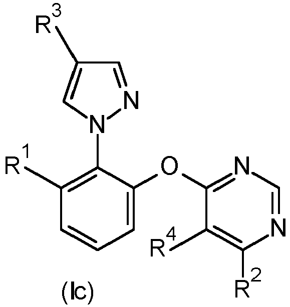 wherein R¹ is selected from the group consisting of hydrogen, fluoro, chloro and bromo, R² is methyl or methoxy, R³ is CF₃ or CF₂H and R⁴ is hydrogen or -CN, preferably hydrogen.

Compounds of Formula (I) may contain asymmetric centres and may be present as a single enantiomer, pairs of enantiomers in any proportion or, where more than one asymmetric centre are present, contain diastereoisomers in all possible ratios. Typically one of the enantiomers has enhanced biological activity compared to the other possibilities.

The present invention also provides agronomically acceptable salts of compounds of Formula (I). Salts that the compounds of Formula (I) may form with amines, including primary, secondary and tertiary amines (for example ammonia, dimethylamine and triethylamine), alkali metal and alkaline earth metal bases, transition metals or quaternary ammonium bases are preferred.

The compounds of Formula (I) according to the invention can be used as herbicides by themselves, but they are generally formulated into herbicidal compositions using formulation adjuvants, such as carriers, solvents and surface-active agents (SAA). Thus, the present invention further provides a herbicidal composition comprising a herbicidal compound according to any one of the previous claims and an agriculturally acceptable formulation adjuvant. The composition can be in the form of concentrates which are diluted prior to use, although ready-to-use compositions can also be made. The final dilution is usually made with water, but can be made instead of, or in addition to, water, with, for example, liquid fertilisers, micronutrients, biological organisms, oil or solvents.

The herbicidal compositions generally comprise from 0.1 to 99 % by weight, especially from 0.1 to 95 % by weight, compounds of Formula I and from 1 to 99.9 % by weight of a formulation adjuvant which preferably includes from 0 to 25 % by weight of a surface-active substance.

The compositions can be chosen from a number of formulation types. These include an emulsion concentrate (EC), a suspension concentrate (SC), a suspoemulsion (SE), a capsule suspension (CS), a water dispersible granule (WG), an emulsifiable granule (EG), an emulsion, water in oil (EO), an emulsion, oil in water (EW), a micro-emulsion (ME), an oil dispersion (OD), an oil miscible flowable (OF), an oil miscible liquid (OL), a soluble concentrate (SL), an ultra-low volume suspension (SU), an ultra-low volume liquid (UL), a technical concentrate (TK), a dispersible concentrate (DC), a soluble powder (SP), a wettable powder (WP) and a soluble granule (SG). The formulation type chosen in any instance will depend upon the particular purpose envisaged and the physical, chemical and biological properties of the compound of Formula (I).

Soluble powders (SP) may be prepared by mixing a compound of Formula (I) with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of Formula (I) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of Formula (I) and one or more powdered solid diluents or carriers, or from preformed blank granules by absorbing a compound of Formula (I) (or a solution thereof, in a suitable agent) in a porous granular material (such as pumice, attapulgite clays, fuller's earth, kieselguhr, diatomaceous earths or ground corn cobs) or by adsorbing a compound of Formula (I) (or a solution thereof, in a suitable agent) on to a hard core material (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of Formula (I) in water or an organic solvent, such as a ketone, alcohol or glycol ether. These solutions may contain a surface active agent (for example to improve water dilution or prevent crystallisation in a spray tank).

Emulsifiable concentrates (EC) or oil-in-water emulsions (EW) may be prepared by dissolving a compound of Formula (I) in an organic solvent (optionally containing one or more wetting agents, one or more emulsifying agents or a mixture of said agents). Suitable organic solvents for use in ECs include aromatic hydrocarbons (such as alkylbenzenes or alkylnaphthalenes, exemplified by SOLVESSO 100, SOLVESSO 150 and SOLVESSO 200; SOLVESSO is a Registered Trade Mark), ketones (such as cyclohexanone or methylcyclohexanone) and alcohols (such as benzyl alcohol, furfuryl alcohol or butanol), N-alkylpyrrolidones (such as N-methylpyrrolidone or N-octylpyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment.

Preparation of an EW involves obtaining a compound of Formula (I) either as a liquid (if it is not a liquid at room temperature, it may be melted at a reasonable temperature, typically below 70°C) or in solution (by dissolving it in an appropriate solvent) and then emulsifying the resultant liquid or solution into water containing one or more SAAs, under high shear, to produce an emulsion. Suitable solvents for use in EWs include vegetable oils, chlorinated hydrocarbons (such as chlorobenzenes), aromatic solvents (such as alkylbenzenes or alkyl naphthalenes) and other appropriate organic solvents which have a low solubility in water.

Microemulsions (ME) may be prepared by mixing water with a blend of one or more solvents with one or more SAAs, to produce spontaneously a thermodynamically stable isotropic liquid formulation. A compound of Formula (I) is present initially in either the water or the solvent/SAA blend. Suitable solvents for use in MEs include those hereinbefore described for use in in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of Formula (I). SCs may be prepared by ball or bead milling the solid compound of Formula (I) in a suitable medium, optionally with one or more dispersing agents, to produce a fine particle suspension of the compound. One or more wetting agents may be included in the composition and a suspending agent may be included to reduce the rate at which the particles settle. Alternatively, a compound of Formula (I) may be dry milled and added to water, containing agents hereinbefore described, to produce the desired end product.

Aerosol formulations comprise a compound of Formula (I) and a suitable propellant (for example n-butane). A compound of Formula (I) may also be dissolved or dispersed in a suitable medium (for example water or a water miscible liquid, such as n-propanol) to provide compositions for use in non-pressurised, hand-actuated spray pumps.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of Formula (I) and, optionally, a carrier or diluent therefor. The polymeric shell may be produced by either an interfacial polycondensation reaction or by a coacervation procedure. The compositions may provide for controlled release of the compound of Formula (I) and they may be used for seed treatment. A compound of Formula (I) may also be formulated in a biodegradable polymeric matrix to provide a slow, controlled release of the compound.

The composition may include one or more additives to improve the biological performance of the composition, for example by improving wetting, retention or distribution on surfaces; resistance to rain on treated surfaces; or uptake or mobility of a compound of Formula (I). Such additives include surface active agents (SAAs), spray additives based on oils, for example certain mineral oils or natural plant oils (such as soy bean and rape seed oil), modified plant oils such as methylated rape seed oil (MRSO), and blends of these with other bio-enhancing adjuvants (ingredients which may aid or modify the action of a compound of Formula (I).

Wetting agents, dispersing agents and emulsifying agents may be SAAs of the cationic, anionic, amphoteric or non-ionic type.

Suitable SAAs of the cationic type include quaternary ammonium compounds (for example cetyltrimethyl ammonium bromide), imidazolines and amine salts.

Suitable anionic SAAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butyl naphthalene sulphonate and mixtures of sodium di-isopropyl- and tri-isopropyl-naphthalene sulphonates), ether sulphates, alcohol ether sulphates (for example sodium laureth-3-sulphate), ether carboxylates (for example sodium laureth-3-carboxylate), phosphate esters (products from the reaction between one or more fatty alcohols and phosphoric acid (predominately mono-esters) or phosphorus pentoxide (predominately di-esters), for example the reaction between lauryl alcohol and tetraphosphoric acid; additionally these products may be ethoxylated), sulphosuccinamates, paraffin or olefine sulphonates, taurates, lignosulphonates and phosphates / sulphates of tristyrylphenols.

Suitable SAAs of the amphoteric type include betaines, propionates and glycinates.

Suitable SAAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); lecithins and sorbitans and esters thereof, alkyl polyglycosides and tristyrylphenols.

Suitable suspending agents include hydrophilic colloids (such as polysaccharides, polyvinylpyrrolidone or sodium carboxymethylcellulose) and swelling clays (such as bentonite or attapulgite).

The herbicidal compounds of present invention can also be used in mixture with one or more additional herbicides and/or plant growth regulators. Examples of such additional herbicides or plant growth regulators include acetochlor, acifluorfen (including acifluorfen-sodium), aclonifen, ametryn, amicarbazone, aminopyralid, aminotriazole, atrazine, bensulfuron (including bensulfuron-methyl), bentazone, bicyclopyrone, bilanafos, bispyribac-sodium, bixlozone, bromacil, bromoxynil, butachlor, butafenacil, carfentrazone (including carfentrazone-ethyl), cloransulam (including cloransulam-methyl), chlorimuron (including chlorimuron-ethyl), chlorotoluron, chlorsulfuron, cinmethylin, clacyfos, clethodim, clodinafop (including clodinafop-propargyl), clomazone, clopyralid, cyclopyranil, cyclopyrimorate, cyclosulfamuron, cyhalofop (including cyhalofop-butyl), 2,4-D (including the choline salt and 2-ethylhexyl ester thereof), 2,4-DB, desmedipham, dicamba (including the aluminium, aminopropyl, bis-aminopropylmethyl, choline, dichloroprop, diglycolamine, dimethylamine, dimethylammonium, potassium and sodium salts thereof) diclosulam, diflufenican, diflufenzopyr, dimethachlor, dimethenamid-P, diquat dibromide, diuron, ethalfluralin, ethofumesate, fenoxaprop (including fenoxaprop-P-ethyl), fenoxasulfone, fenquinotrione, fentrazamide, flazasulfuron, florasulam, florpyrauxifen (including florpyraxifen-benzyl), fluazifop (including fluazifop-P-butyl), flucarbazone (including flucarbazone-sodium), flufenacet, flumetsulam, flumioxazin, flupyrsulfuron (including flupyrsulfuron-methyl-sodium), fluroxypyr (including fluroxypyr-meptyl), fomesafen, foramsulfuron, glufosinate (including the ammonium salt thereof), glyphosate (including the diammonium, isopropylammonium and potassium salts thereof), halauxifen (including halauxifen-methyl), haloxyfop (including haloxyfop-methyl), hexazinone, hydantocidin, imazamox, imazapic, imazapyr, imazethapyr, indaziflam, iodosulfuron (including iodosulfuron-methyl-sodium), iofensulfuron (including iofensulfuron-sodium), ioxynil, isoproturon, isoxaflutole, lancotrione, MCPA, MCPB, mecoprop-P, mesosulfuron (including mesosulfuron-methyl), mesotrione, metamitron, metazachlor, methiozolin, metolachlor, metosulam, metribuzin, metsulfuron, napropamide, nicosulfuron, norflurazon, oxadiazon, oxasulfuron, oxyfluorfen, paraquat dichloride, pendimethalin, penoxsulam, phenmedipham, picloram, pinoxaden, pretilachlor, primisulfuron-methyl, propanil, propaquizafop, propyrisulfuron, propyzamide, prosulfocarb, prosulfuron, pyraclonil, pyraflufen (including pyraflufen-ethyl), pyrasulfotole, pyridate, pyriftalid, pyrimisulfan, pyroxasulfone, pyroxsulam, quinclorac, quinmerac, quizalofop (including quizalofop-P-ethyl and quizalofop-P-tefuryl), rimsulfuron, saflufenacil, sethoxydim, simazine, S-metalochlor, sulfentrazone, sulfosulfuron, tebuthiuron, tefuryltrione, tembotrione, terbuthylazine, terbutryn, thiencarbazone, thifensulfuron, tiafenacil, tolpyralate, topramezone, tralkoxydim, triafamone, triallate, triasulfuron, tribenuron (including tribenuron-methyl), triclopyr, trifloxysulfuron (including trifloxysulfuron-sodium), trifludimoxazin, trifluralin, triflusulfuron, 4-hydroxy-1-methoxy-5-methyl-3-[4-(trifluoromethyl)-2-pyridyl]imidazolidin-2-one, 4-hydroxy-1,5-dimethyl-3-[4-(trifluoromethyl)-2-pyridyl]imidazolidin-2-one, 5-ethoxy-4-hydroxy-1-methyl-3-[4-(trifluoromethyl)-2-pyridyl]imidazolidin-2-one, 4-hydroxy-1-methyl-3-[4-(trifluoromethyl)-2-pyridyl]imidazolidin-2-one, 4-hydroxy-1,5-dimethyl-3-[1-methyl-5-(trifluoromethyl)pyrazol-3-yl]imidazolidin-2-one, (4R)1-(5-tert-butylisoxazol-3-yl)-4-ethoxy-5-hydroxy-3-methyl-imidazolidin-2-one, 3-[2-(3,4-dimethoxyphenyl)-6-methyl-3-oxo-pyridazine-4-carbonyl]bicyclo[3.2.1]octane-2,4-dione, 2-[2-(3,4-dimethoxyphenyl)-6-methyl-3-oxo-pyridazine-4-carbonyl]-5-methyl-cyclohexane-1,3-dione, 2-[2-(3,4-dimethoxyphenyl)-6-methyl-3-oxo-pyridazine-4-carbonyl]cyclohexane-1,3-dione, 2-[2-(3,4-dimethoxyphenyl)-6-methyl-3-oxo-pyridazine-4-carbonyl]-5,5-dimethyl-cyclohexane-1,3-dione, 6-[2-(3,4-dimethoxyphenyl)-6-methyl-3-oxo-pyridazine-4-carbonyl]-2,2,4,4-tetramethyl-cyclohexane-1,3,5-trione, 2-[2-(3,4-dimethoxyphenyl)-6-methyl-3-oxo-pyridazine-4-carbonyl]-5-ethyl-cyclohexane-1,3-dione, 2-[2-(3,4-dimethoxyphenyl)-6-methyl-3-oxo-pyridazine-4-carbonyl]-4,4,6,6-tetramethyl-cyclohexane-1,3-dione, 2-[6-cyclopropyl-2-(3,4-dimethoxyphenyl)-3-oxo-pyridazine-4-carbonyl]-5-methyl-cyclohexane-1,3-dione, 3-[6-cyclopropyl-2-(3,4-dimethoxyphenyl)-3-oxo-pyridazine-4-carbonyl]bicyclo[3.2.1]octane-2,4-dione, 2-[6-cyclopropyl-2-(3,4-dimethoxyphenyl)-3-oxo-pyridazine-4-carbonyl]-5,5-dimethyl-cyclohexane-1,3-dione, 6-[6-cyclopropyl-2-(3,4-dimethoxyphenyl)-3-oxo-pyridazine-4-carbonyl]-2,2,4,4-tetramethyl-cyclohexane-1,3,5-trione, 2-[6-cyclopropyl-2-(3,4-dimethoxyphenyl)-3-oxo-pyridazine-4-carbonyl]cyclohexane-1,3-dione, 4-[2-(3,4-dimethoxyphenyl)-6-methyl-3-oxo-pyridazine-4-carbonyl]-2,2,6,6-tetramethyl-tetrahydropyran-3,5-dione and 4-[6-cyclopropyl-2-(3,4-dimethoxyphenyl)-3-oxo-pyridazine-4-carbonyl]-2,2,6,6-tetramethyl-tetrahydropyran-3,5-dione.

The mixing partners of the compound of Formula (I) may also be in the form of esters or salts, as mentioned e.g. in The Pesticide Manual, Sixteenth Edition, British Crop Protection Council, 2012.

The compound of Formula (I) can also be used in mixtures with other agrochemicals such as fungicides, nematicides or insecticides, examples of which are given in The Pesticide Manual.

The mixing ratio of the compound of Formula (I) to the mixing partner is preferably from 1: 100 to 1000:1.

The mixtures can advantageously be used in the above-mentioned formulations (in which case "active ingredient" relates to the respective mixture of compound of Formula (I) with the mixing partner).

The compounds or mixtures of the present invention can also be used in combination with one or more herbicide safeners. Examples of such safeners include benoxacor, cloquintocet (including cloquintocet-mexyl), cyprosulfamide, dichlormid, fenchlorazole (including fenchlorazole-ethyl), fenclorim, fluxofenim, furilazole, isoxadifen (including isoxadifen-ethyl), mefenpyr (including mefenpyr-diethyl), metcamifen and oxabetrinil.

Particularly preferred are mixtures of a compound of Formula (I) with cyprosulfamide, isoxadifen-ethyl, cloquintocet-mexyl and/or N-(2-methoxybenzoyl)-4-[(methyl-aminocarbonyl)amino]benzenesulfonamide.

The safeners of the compound of Formula (I) may also be in the form of esters or salts, as mentioned e.g. in The Pesticide Manual, 16th Edition (BCPC), 2012. The reference to cloquintocet-mexyl also applies to a lithium, sodium, potassium, calcium, magnesium, aluminium, iron, ammonium, quaternary ammonium, sulfonium or phosphonium salt thereof as disclosed in WO 02/34048.

Preferably the mixing ratio of compound of Formula (I) to safener is from 100:1 to 1:10, especially from 20:1 to 1:1.

The present invention still further provides a method of controlling weeds at a locus said method comprising application to the locus of a weed controlling amount of a composition comprising a compound of Formula (I). Moreover, the present invention may further provide a method of selectively controlling weeds at a locus comprising crop plants and weeds, wherein the method comprises application to the locus of a weed controlling amount of a composition according to the present invention. 'Controlling' means killing, reducing or retarding growth or preventing or reducing germination. It is noted that the compounds of the present invention show much improved selectivity compared to know, structurally similar compounds. Generally the plants to be controlled are unwanted plants (weeds). 'Locus' means the area in which the plants are growing or will grow. The application may be applied to the locus pre-emergence and/or postemergence of the crop plant. Some crop plants may be inherently tolerant to herbicidal effects of compounds of Formula (I). Preferred crop plants include maize, wheat, barley and rice.

The rates of application of compounds of Formula I may vary within wide limits and depend on the nature of the soil, the method of application (pre- or post-emergence; seed dressing; application to the seed furrow; no tillage application etc.), the crop plant, the weed(s) to be controlled, the prevailing climatic conditions, and other factors governed by the method of application, the time of application and the target crop. The compounds of Formula I according to the invention are generally applied at a rate of from 10 to 2500 g/ha, especially from 25 to 1000 g/ha, more especially from 25 to 250 g/ha.

The application is generally made by spraying the composition, typically by tractor mounted sprayer for large areas, but other methods such as dusting (for powders), drip or drench can also be used.

Crop plants are to be understood as also including those crop plants which have been rendered tolerant to other herbicides or classes of herbicides (e.g. ALS-, GS-, EPSPS-, PPO-, HPPD-, -PDS and ACCase-inhibitors) by conventional methods of breeding or by genetic engineering. An example of a crop that has been rendered tolerant to imidazolinones, e.g. imazamox, by conventional methods of breeding is Clearfield^{®} summer rape (canola). Examples of crops that have been rendered tolerant to herbicides by genetic engineering methods include e.g. glyphosate- and glufosinate-resistant maize varieties commercially available under the trade names RoundupReady^{®} and LibertyLink^{®}.

Crop plants are also to be understood as being those which have been rendered resistant to harmful insects by genetic engineering methods, for example Bt maize (resistant to European corn borer), Bt cotton (resistant to cotton boll weevil) and also Bt potatoes (resistant to Colorado beetle). Examples of Bt maize are the Bt 176 maize hybrids of NK^{®} (Syngenta Seeds). The Bt toxin is a protein that is formed naturally by *Bacillus thuringiensis* soil bacteria. Examples of toxins, or transgenic plants able to synthesise such toxins, are described in EP-A-451 878, EP-A-374 753, WO 93/07278, WO 95/34656, WO 03/052073 and EP-A-427 529. Examples of transgenic plants comprising one or more genes that code for an insecticidal resistance and express one or more toxins are KnockOut^{®} (maize), Yield Gard^{®} (maize), NuCOTIN33B^{®} (cotton), Bollgard^{®} (cotton), NewLeaf^{®} (potatoes), NatureGard^{®} and Protexcta^{®}. Plant crops or seed material thereof can be both resistant to herbicides and, at the same time, resistant to insect feeding ("stacked" transgenic events). For example, seed can have the ability to express an insecticidal Cry3 protein while at the same time being tolerant to glyphosate.

Crop plants are also to be understood to include those which are obtained by conventional methods of breeding or genetic engineering and contain so-called output traits (e.g. improved storage stability, higher nutritional value and improved flavour).

The compositions can be used to control unwanted plants (collectively, 'weeds'). The weeds to be controlled may be both monocotyledonous species, for example *Agrostis, Alopecurus, Avena, Brachiaria, Bromus, Concerns, Cyperus, Digitaria, Echinochloa, Eleusine, Lolium, Monochoria, Rottboellia, Sagittaria, Scirpus, Setaria and Sorghum,* and dicotyledonous species, for example *Abutilon, Amaranthus, Ambrosia, Chenopodium, Chrysanthemum, Conyza, Galium, Ipomoea, Nasturtium, Sida, Sinapis, Solanum, Stellaria, Veronica, Viola and Xanthium.*

In a further aspect of the present invention there is provided the use of a compound of Formula (I) as defined herein as a herbicide.

The compounds of the present invention can be prepared according to the following schemes.

### Processes for preparation of compounds of Formula (I)

Compounds of the invention can be prepared by reaction of the relevant phenol with chloropyrimidines. The desired chloropyrimidines can be prepared from the corresponding dichloropyrimidine, introducing the required R² substituent by aromatic substitution reactions with the relevant nucleophiles or Pd catalysed cross-coupling chemistry (Scheme 1, Path A). Alternatively the steps can be interchanged (Scheme 1, Path B).

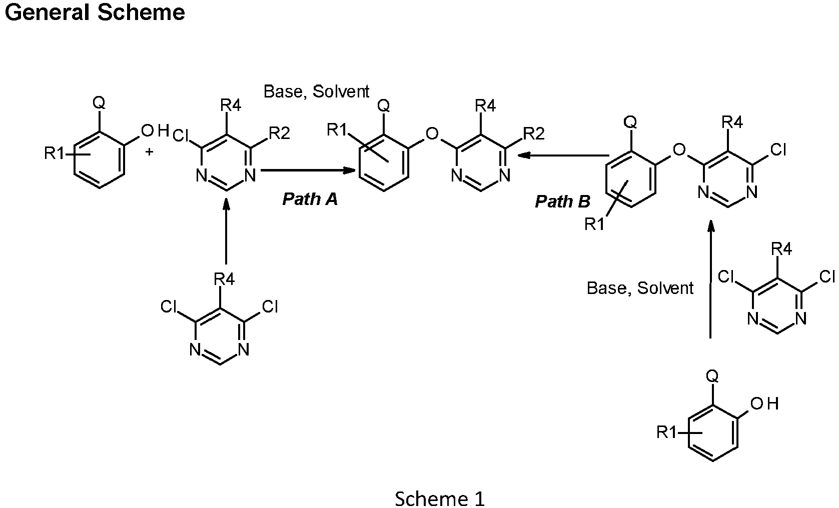

### Synthesis of 2-[3-(difluoromethyl)isoxazol-5-yl]-3-fluoro-phenol

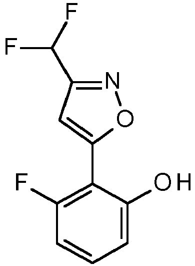

2-[3-(Difluoromethyl)isoxazol-5-yl]-3-fluoro-phenol was prepared according to procedures described in WO2015/108779. Phenols with alternative R¹ substituents can be prepared by analogous methods.

### Synthesis of 3-(2-fluoro-6-hydroxy-phenyl)-5-(trifluoromethyl)-4H-isoxazol-5-ol

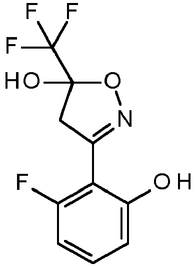

3-(2-fluoro-6-hydroxy-phenyl)-5-(trifluoromethyl)-4H-isoxazol-5-ol was prepared according to procedures described in WO2015/108779.

### Synthesis of 3-fluoro-2-[5-(trifluoromethyl)isoxazol-3-yl]phenol

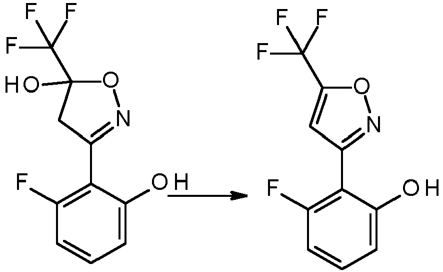

To a stirred solution of 3-(2-fluoro-6-hydroxy-phenyl)-5-(trifluoromethyl)-4H-isoxazol-5-ol (20.0 g, 75.4 mmol) in toluene (150 mL) was added p-toluene sulphonic acid monohydrate (1.59 g, 9.05 mmol). The reaction was heated at reflux for 2h with azeotropic removal of water then cooled to RT and evaporated to dryness under reduced pressure. The residue was dissolved in CH₂Cl₂ (500 mL) and added gradually to a stirred solution of saturated aq. NaHCO₃ (200 mL). The organic phase was separated and washed with H₂O then brine and evaporated to dryness under reduced pressure to leave the desired product (17.41 g, 93%) as an off white solid which was used without further purification.

¹H NMR (400 MHz, CDCl₃) δ 9.54 (s, 1H), 7.35 (m, 2H), 6.94 (d, 1H), 6.76 (t, 1H).

The following non-limiting examples provide specific synthesis methods for representative compounds of the present invention, as referred to in the Tables below.

### Example 1: Synthesis of 3-(difluoromethyl)-5-[2-fluoro-6-(6-methoxypyrimidin-4-yl)oxy-phenyl]isoxazole

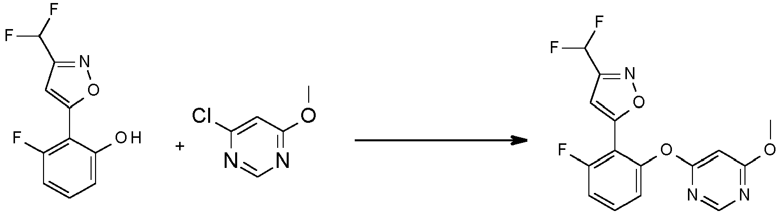

To a solution of 2-[3-(difluoromethyl)isoxazol-5-yl]-3-fluoro-phenol (0.60 g, 2.6 mmol) stirred and dissolved in *N*,*N*-dimethylformamide (26 mL) was added potassium carbonate (1.8 g, 13 mmol) and 4-chloro-6-methoxy-pyrimidine (1.1 g, 7.9 mmol). The mixture was heated at 75°C for 40h. The reaction mixture was then allowed to cool and concentrated to remove the bulk of DMF. The residual material was diluted with water (15 mL) and extracted with ether (3 x 20 mL). The organic extracts were combined, washed with water (2 x 10 mL) and separated. The collected organics were adsorbed onto granular celite and purified by chromatography, gradient elution with EtOAc/cyclohexane to afford the desired product (400 mg, 45%).

¹H NMR: (400MHz, CDCl₃) δ 8.38 (d, 1H), 7.53 (dt, 1H), 7.18 (ddd, 1H), 7.09 (dt, 1H), 6.90 - 6.63 (t, 1H), 6.82 (d, 1H), 6.31 (d, 1H), 3.99 (s, 3H).

### Example 2 Synthesis of 3-[2-(6-bromopyrimidin-4-yl)oxy-6-fluoro-phenyl]-5-(trifluoromethyl)isoxazole

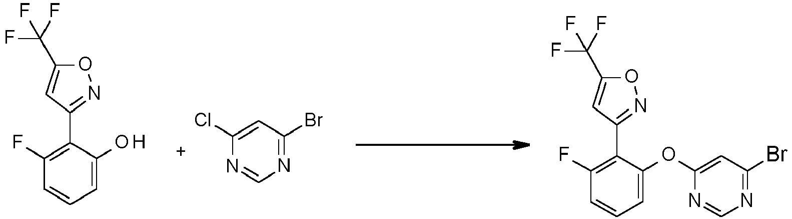

To a solution of 3-fluoro-2-[5-(trifluoromethyl)isoxazol-3-yl]phenol (0.10 g, 0.40 mmol) stirred and dissolved in *N*,*N*-dimethylformamide (4.0 mL) was added potassium carbonate (0.28 g, 2.0 mmol) and 4,6-dibromopyrimidine (0.14 g, 0.61 mmol) and the light orange-brown mixture was stirred at for 42h at ambient temperature.The reaction mixture was then diluted with water (8 mL) and extracted with Et₂O (3 × 8 mL). The organic extracts were combined, washed with water (2 × 10 mL) and separated. The collected organics were adsorbed onto granular celite and purified by chromatography, gradient elution with EtOAc/cyclohexane to afford the desired product (7mg, 4%).

¹H NMR: (400MHz, CDCl₃) δ 8.46 (d, 1H), 7.58 (dt, 1H), 7.26 - 7.21 (m, 1H), 7.20 (d, 1H), 7.12 (dt, 1H), 7.03 - 6.96 (m, 1H)

### Example 3: 3-(6-methoxypyrimidin-4-yl)oxy-2-[4-(trifluoromethyl)pyrazol-1-yl]benzonitrile

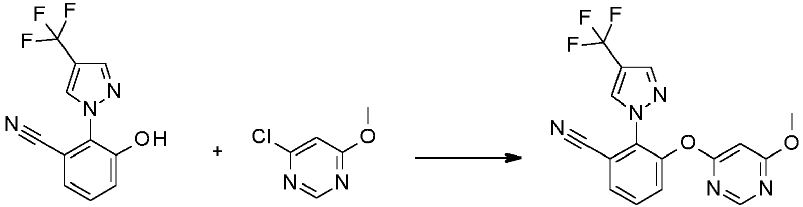

To a solution of 3-hydroxy-2-[4-(trifluoromethyl)pyrazol-1-yl]benzonitrile (0.200 g, 0.790 mmol) and 4-chloro-6-methoxy-pyrimidine (0.171 g, 1.18 mmol) in *N,N-*dimethylformamide (7.90 mL) in a microwave vial was added potassium carbonate (0.546 g, 3.95 mmol). The solution was heated at 130°C for 2 hours under microwave irradiation. The reaction mixture was poured into water and diluted with ethyl acetate resulting in formation of a yellow mixture. The aqueous phase was acidified to pH 1 (2M HCl). The phases were separated and the aqueous phase was extracted into ethyl acetate (2X10 mL). The combined organic extracts were washed with brine, dried over magnesium sulfate, filtered and concentrated to afford a brown liquid. Purification by RPHPLC afforded the desired product as a white solid (0.108 g, 38%). ¹H NMR (400 MHz, CDCl₃) δ 8.32 (d, 1H), 7.95 (s, 1H), 7.90 (s, 1H), 7.75 (dd, H), 7.64 (t, 1H), 7.59 - 7.56 (m, 1H), 6.20 (d, 1H), 3.98 (s, 3H)

### Synthesis of 3-hydroxy-2-[4-(trifluoromethyl)pyrazol-1-yl]benzonitrile

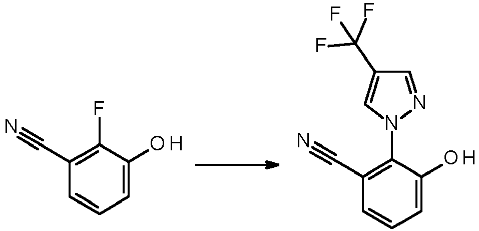

To a solution of 2-fluoro-3-hydroxy-benzonitrile (1.0 g, 7.3 mmol) and 4-(trifluoromethyl)-1H-pyrazole (1.1 g, 8.0 mmol) in N,N-dimethylacetamide (15 mL) under a nitrogen atmosphere was added powdered K₂CO₃ (3.1 g, 22 mmol). The resultant mixture was heated at 150°C for 22 hours. The reaction mixture was allowed to cool to RT, diluted with water and extracted with EtOAc (x 4). The combined organic extracts were washed with brine, dried over MgSO₄ and evaporated to dryness to give an orange liquid. The crude product was purified by flash chromatography on silica gel using a gradient of 20 to 40% ethyl acetate in cyclohexane as eluent to give the desired product (594mg, 32%) as a yellow solid.

¹H NMR (400 MHz, CDCl₃) δ 8.64 (s, 1H), 8.07 (s, 1H), 7.42 - 7.35 (m, 3H).

### Example 4: Synthesis of 5-[2-(6-chloro-5-fluoro-pyrimidin-4-yl)oxy-6-fluorophenyl]-3-(difluoromethyl)isoxazole

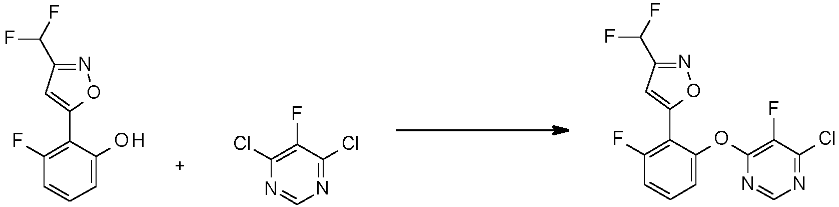

To a solution of 2-[3-(difluoromethyl)isoxazol-5-yl]-3-fluoro-phenol (0.1 g, 0.44 mmol) stirred and dissolved in *N*,*N*-dimethylformamide (4.5 mL) was added potassium carbonate (0.3 g, 2.2 mmol) and 4,6-dichloro-5-fluoro-pyrimidine (0.11 g, 0.65 mmol). After 4h, the mixture was diluted with water (3 mL) and extracted with ether (3 x 8 mL). The organic extracts were combined, washed with water (2 x 10 mL) and separated. The collected organics were adsorbed onto granular celite and purified by chromatography, gradient elution with EtOAc/cyclohexane to afford the desired product (115 mg, 74%).

¹H NMR: (400MHz, CDCl₃) δ 8.24 (s, 1H), 7.59 (dt, 1H), 7.27 (dt, *J* = 1.0, 9.2 Hz, 1H), 7.17 (td, 1H), 6.91 (s, 1H), 6.90 - 6.64 (t, 1H)

### Example 5: Synthesis of 3-(difluoromethyl)-5-[2-fluoro-6-(5-fluoro-6-methoxy-pyrimidin-4-yl)oxy-phenyl]isoxazole

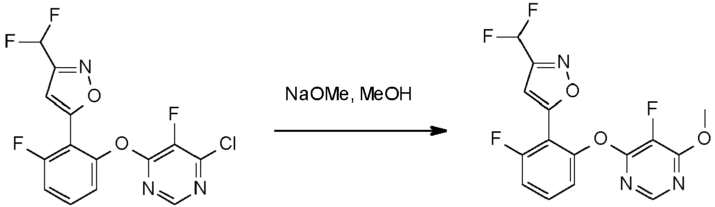

To a solution of 5-[2-(6-chloro-5-fluoro-pyrimidin-4-yl)oxy-6-fluoro-phenyl]-3-(difluoromethyl)isoxazole (0.050 g, 0.14 mmol) in methanol (5 mL) stirred and cooled to 0-5°C in an ice bath was added sodium methoxide (0.50M in MeOH) (0.8 mL, 0.42 mmol). The colourless solution was stirred at room temp for 3 hours, then left to stand for 48h. The reaction mixture was concentrated to remove the bulk of solvent, and the residue quenched by the addition of water then acidified with dil HCl. A white precipitate was filtered off, washing through with water then drying under suction giving a white powder. Purification by RPHPLC afforded the desired product (2.3mg, 4.7%).

¹H NMR: (400MHz, CDCl₃) δ 8.07 (s, 1H), 7.55 (dt, 1H), 7.21 (ddd, 1H), 7.15 - 7.11 (m, 1H), 6.93 - 6.62 (t, 1H), 6.87 (d, 1H), 4.09 (s, 3H).

### Example 6: Synthesis of 5-(difluoromethyl)-2-[2-fluoro-6-(6-methoxypyrimidin-4-yl)oxy-phenyl]oxazole

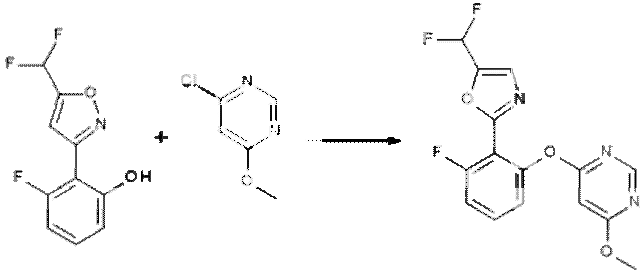

To a solution of 2-[5-(difluoromethyl)isoxazol-3-yl]-3-fluoro-phenol (0.100 g, 0.436 mmol) stirred and dissolved in *N*,*N*-dimethylformamide (4.5 mL) was added potassium carbonate (0.091 g, 0.655 mmol) and 4-chloro-6-methoxy-pyrimidine (0.095 g, 0.655 mmol), the mixture being stirred and heated to 70°C for 40h. The reaction mixture was allowed to cool and was diluted with water (5 mL) and was extracted with Et2O (3 x 8 mL). The organic extracts were combined, washed with water (2 x 10 mL) and separated and the collected organics were concentrated to give crude material as a brown gum. This was dissolved in 1 mL 10% MeOH in DMSO and purified by RPHPLC to afford the product as a pale-yellow solid (46.7 mg, 32%).

¹H NMR: (400MHz, CDCl₃) δ 8.35 (s, 1H), 7.55 (dt, 1H), 7.40 (t, 1H), 7.22 - 7.14 (m, 1H), 7.11 (d, 1H), 6.79 - 6.52 (t, 1H), 6.26 (d, 1H), 3.98 (s, 3H).

### Example 7: Synthesis of 4-[3-bromo-2-(5,5,5-trifluoropentyl)phenoxy]-6-methoxy-pyrimidine

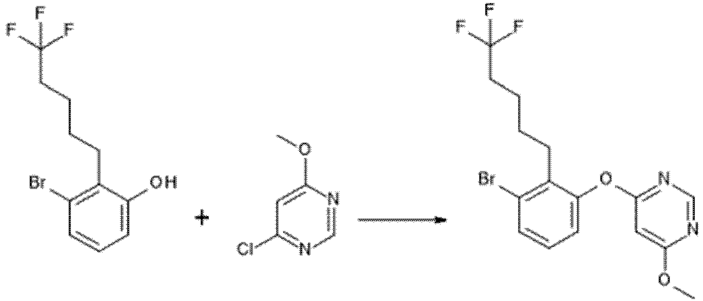

4-Chloro-6-methoxypyrimidine (0.046 g, 0.32 mmol) and potassium carbonate (0.073 g, 0.53 mmol) were stirred in *N*,*N*-dimethylformamide (4 mL) at ambient temperature to which 3-bromo-2-(5,5,5-trifluoropentyl)phenol (0.078 g, 0.26 mmol) was added in one portion. The reaction was stirred at 80 °C until completion. The reaction mixture was concentrated onto celite and purified by column chromatography on silica gel using a gradient of 0-10% EtOAc/cyclohexane afforded 4-[3-bromo-2-(5,5,5-trifluoropentyl)phenoxy]-6-methoxy-pyrimidine (0.100 g, 94%) as a colourless gum.

¹H NMR: (400MHz, CDCl₃) δ 8.45 (s, 1H), 7.48 (dd, 1H), 7.13 (t, 1H), 7.01 (dd, 1H), 6.16 (d, 1H), 3.99 (s, 3H), 2.71 (br t, 2H), 2.13-1.99 (m, 2H), 1.62-1.57 (m, 4H).

### Example 8: Synthesis of 3-bromo-2-(5,5,5-trifluoropentyl)phenol

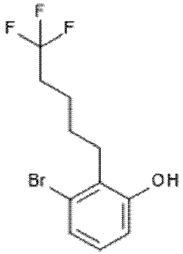

3-Bromo-2-(5,5,5-trifluoropentyl)phenol was prepared according to procedures described in WO2016/196606. Phenols with alternative R1 substituents can be prepared by analogous methods.

**Table 1 - Examples of herbicidal compounds of the present invention.**

| **COMPOUND** | **STRUCTURE** | **¹H NMR (400MHz, CDCl₃)** |
|---|---|---|
| 1.001 | 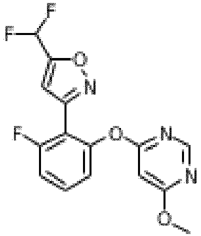 | 8.38 (s, 1H), 7.52 (dt, 1H), 7.17 (ddd, 1H), 7.08 (d, 1H), 6.81 (d, 1H), 6.88-6.62 (t, 1H), 6.22 (d, 1H), 3.98 (s, 3H) |
| 1.002 | 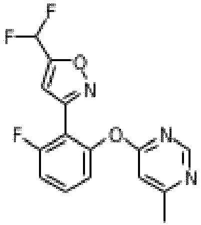 | 8.67 (s, 1H), 7.56 (dt, 1H), 7.22 (ddd, 1H), 7.10 (d, 1H), 6.88 - 6.62 (t, 1H), 6.87 (s, 1H), 6.86-6.83 (m, 1H), 5.40 (br s, 2H), 2.58 (s, 3H) |
| 1.003 | 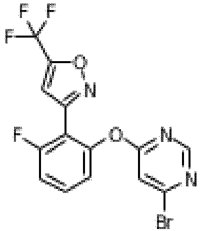 | 8.46 (d, 1H), 7.58 (dt, 1H), 7.26 - 7.21 (m, 1H), 7.20 (d, 1H), 7.12 (dt, 1H), 7.03-6.96 (m, 1H) |
| 1.004 | 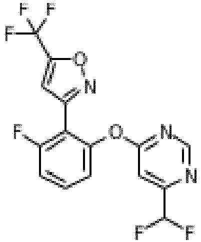 | 8.75 (s, 1H), 7.59 (dt, 1H), 7.26-7.21 (m, 2H), 7.18-7.10 (m, 1H), 7.03-6.97 (m, 1H), 6.77-6.36 (t, 1H) |
| 1.005 | 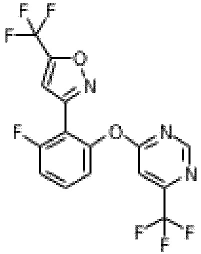 | 8.81 (s, 1H), 7.60 (dt, 1H), 7.33 (d, 1H), 7.29-7.23 (m, 1H), 7.15 (td, 1H), 7.04-7.00 (m, 1H) |
| 1.006 | 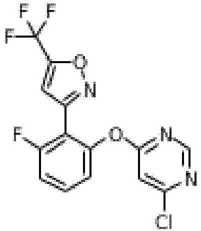 | 8.51 (d, 1H), 7.58 (dt, 1H), 7.25-7.20 (m, 1H), 7.12 (dt, 1H), 7.01 (d, 1H), 7.00 (dd, 1H) |
| 1.007 | 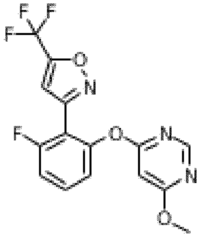 | 8.38 (s, 1H), 7.54 (dt, 1H), 7.2 -7.15 (m, 1H), 7.09 (d, 1H), 6.95 (br.d, 1H), 6.24 (d, 1H), 3.99 (s, 3H) |

**Table 2 - Examples of herbicidal compounds of the present invention.**

| **COMPOUND** | **STRUCTURE** | **¹H NMR (400MHz, CDCl₃)** |
|---|---|---|
| 2.001 | 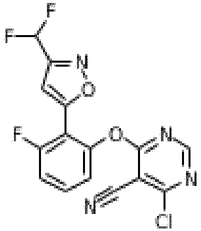 | 8.58 (s, 1H), 7.61 (dt, 1H), 7.31 (ddd, 1H), 7.16 (dd, 1H), 6.95 (d, 1H), 6.92-6.63 (t, 1H) |
| 2.002 | 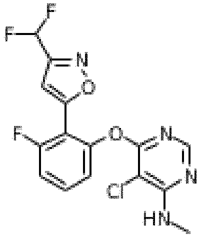 | 8.09 (s, 1H), 7.53 (dt, 1H), 7.17 (ddd, 1H), 7.12 (td, 1H), 6.92-6.62 (t, 1H), 6.87 (d, 1H), 5.47 (br d, 1H), 3.11 (d, 3H) |
| 2.003 | 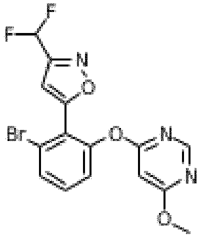 | 8.39 (d, 1H), 7.64 (dd, 1H), 7.44 (t, 1H), 7.22 (dd, 1H), 6.91-6.62 (t, 1H), 6.60 (s, 1H), 6.15 (d, , 1H), 3.97 (s, 3H) |
| 2.004 | 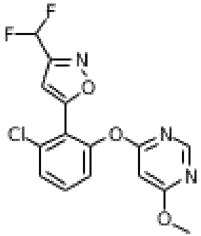 | 8.39 (d, 1H), 7.51 (t, 1H), 7.46 (dd, 1H), 7.18 (dd, 1H), 6.92-6.60 (t, 1H), 6.65 (d, 1H), 6.17 (d, 1H), 3.98 (s, 3H) |
| 2.005 | 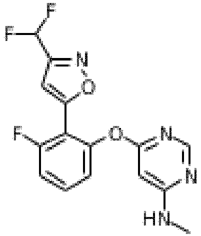 | 8.16 (s, 1H), 7.51 (dt, 1H), 7.14 (ddd, 1H), 7.10-7.06 (m, 1H), 6.93-6.62 (t, 1H), 6.81 (d, 1H), 5.90 (s, 1H), 5.09 (br s, 1H), 2.95 (d, 3H) |
| 2.006 | 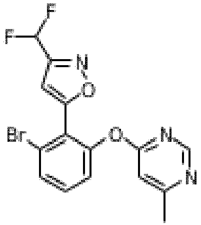 | 8.62 (s, 1H), 7.66 (dd, 1H), 7.46 (t, 1H), 7.24 (dd, 1H), 6.92-6.61 (t, 1H), 6.70 (s, 1H), 6.59 (s, 1H), 2.49 (s, 3H) |
| 2.007 | 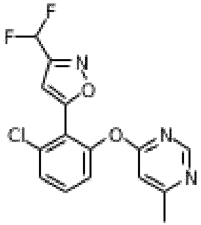 | 8.61 (d, 1H), 7.53 (t, 1H), 7.48 (dd, 1H), 7.21 (dd, 1H), 6.89-6.62 (t, 1H), 6.73 (s, 1H), 6.64 (s, 1H), 2.50 (s, 3H) |
| 2.008 | 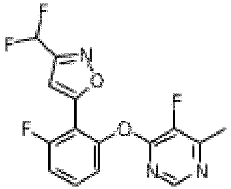 | 8.31 (s, 1H), 7.57 (dt, 1H), 7.26-7.20 (m, 1H), 7.16 (d, 1H), 6.88 (d, 1H), 6.92-6.61 (t, 1H), 2.57 (d, 3H) |
| 2.009 | 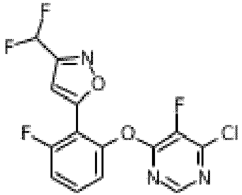 | 8.24 (s, 1H), 7.59 (dt, 1H), 7.27 (dt, 1H), 7.17 (td, 1H), 6.91 (s, 1H), 6.90-6.64 (t, 1H) |
| 2.010 | 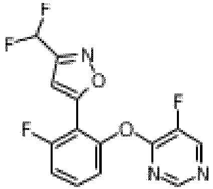 | 8.54 (d, 1H), 8.45 (d, 1H), 7.59 (dt, 1H), 7.26 (dt, 1H), 7.19 (td, 1H), 6.89 (d, 1H), 6.88-6.61 (t, 1H) |
| 2.011 | 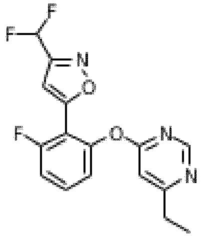 | 8.61 (d, 1H), 7.55 (dt, 1H), 7.23 - 7.16 (m, 1H), 7.11 (d, 1H), 6.89-6.82 (t, 1H), 6.88 (s, 1H), 6.81 (d, 1H), 2.81 (q, 2H), 1.33 (t, 3H) |
| 2.012 | 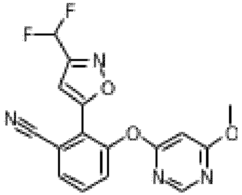 | 8.38-8.34 (m, 1H), 7.77 (dd, 1H), 7.67 (t, 1H), 7.53 (dd, 1H), 6.94-6.63 (m, 1H), 6.91 (s, 1H), 6.31 (d, 1H), 4.00 (s, 3H) |
| 2.013 | 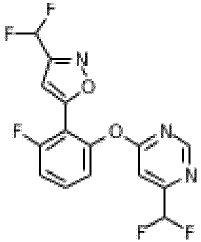 | 8.74 (s, 1H), 7.58 (dt, 1H), 7.34 (s, 1H), 7.25 (dt, 1H), 7.12 (dt, 1H), 6.89-6.62 (t, 1H), 6.85 (d, 1H), 6.71-6.43 (t, 1H) |
| 2.014 | 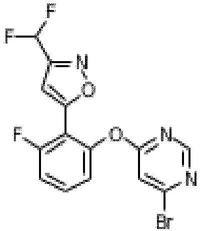 | 8.46 (d, 1H), 7.57 (dt, 1H), 7.28 (d, 1H), 7.26-7.20 (m, 1H), 7.10 (d, 1H), 6.85 (d, 1H), 6.92-6.62 (t, 1H) |
| 2.015 | 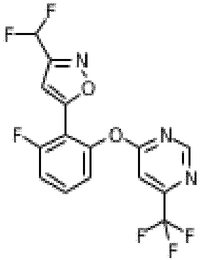 | 8.81 (s, 1H), 7.59 (dt, 1H), 7.40 (d, 1H), 7.29-7.24 (m, 1H), 7.13 (d, 1H), 6.87 (d, 1H), 6.76 (t, 1H) |
| 2.016 | 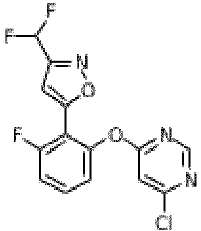 | 8.50 (d, 1H), 7.57 (dt, 1H), 7.26-7.20 (m, 1H), 7.11 (dt, 1H), 7.09 (d, 1H), 6.85 (d, 1H), 6.77 (t, 1H) |
| 2.017 | 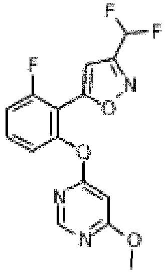 | 8.38 (d, 1H), 7.53 (dt, 1H), 7.18 (ddd, 1H), 7.09 (dt, 1H), 6.90-6.63 (t, 1H), 6.82 (d, 1H), 6.31 (d, 1H), 3.99 (s, 3H) |
| 2.018 | 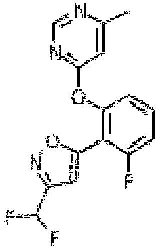 | 8.59 (d, 1H), 7.55 (dt, 1H), 7.20 (ddd, 1H), 7.10 (dt, 1H), 6.90-6.61 (t, 1H), 6.88 (s, 1H), 6.81 (d, 1H), 2.54 (s, 3H) |
| 2.019 | 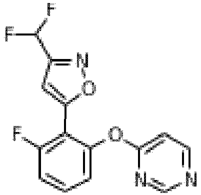 | 8.78-8.44 (m, 2H), 7.67-7.43 (m, 1H), 7.34-7.01 (m, 3H), 6.93-6.54 (m, 2H) |
| 2.020 | 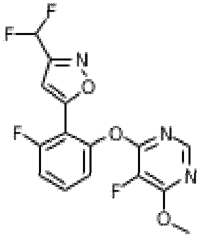 | 8.07 (s, 1H), 7.55 (dt, 1H), 7.21 (ddd, 1H), 7.15-7.11 (m, 1H), 6.93-6.62 (t, 1H), 6.87 (d, 1H), 4.09 (s, 3H) |
| 2.021 | 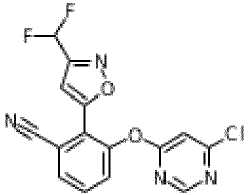 | 8.51 (d, 1H), 7.82 (dd, 1H), 7.72 (t, 1H), 7.56 (dd, 1H), 7.07 (d, 1H), 6.97 (s, 1H), 6.80 (t, 1H) |
| 2.022 | 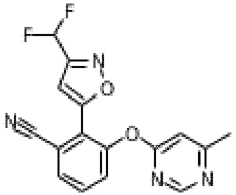 | 8.61 - 8.57 (m, 1H), 7.79 (dd, 1H), 7.69 (t, 1H), 7.56 (dd, 1H), 6.91 (s, 1H), 6.86 (s, 1H), 6.79 (t, 1H), 2.55 (s, 3H) |
| 2.023 | 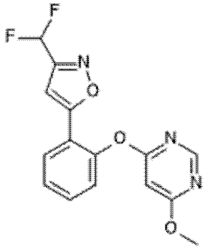 | 8.44 (s, 1H), 8.06 (dd, 1H), 7.58-7.51 (m, 1H), 7.47-7.39 (m, 1H), 7.24 (dd, 1H), 6.89-6.62 (t, 1H), 6.78 (s, 1H), 6.26 (d, 1H), 4.00 (s, 3H) |
| 2.024 | 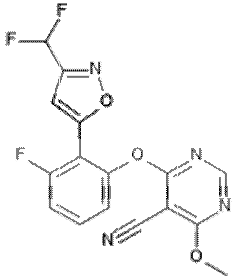 | 8.39 (s, 1H), 7.58 (dt, 1H), 7.30 - 7.22 (m, 1H), 7.17-7.12 (m, 1H), 6.91 (d, 1H), 6.91-6.64 (t, 1H), 4.16 (s, 3H) |

**Table 3 - Examples of herbicidal compounds of the present invention.**

| **COMPOUND** | **STRUCTURE** | **¹H NMR (400MHz, CDCl₃)** |
|---|---|---|
| 3.001 | 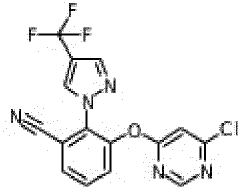 | 8.47 (d, 1H), 7.97 (s, 1H), 7.88 (s, 1H), 7.80 (dd, 1H), 7.68 (t, 1H), 7.62-7.59 (m, 1H), 6.94 (d, 1H) |
| 3.002 | 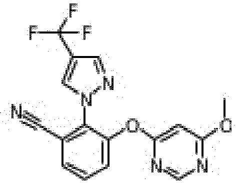 | 8.32 (d, 1H), 7.95 (s, 1H), 7.90 (s, 1H), 7.75 (dd, 1H), 7.64 (t, 1H), 7.59-7.56 (m, 1H), 6.20 (d, 1H), 3.98 (s, 3H) |
| 3.003 | 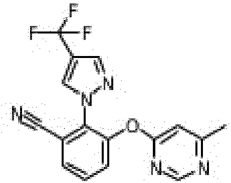 | 8.57-8.53 (m, 1H), 7.94 (s, 1H), 7.88 (s, 1H), 7.77 (dd, 1H), 7.66 (t, 1H), 7.61-7.58 (m, 1H), 6.73 (s, 1H), 2.50 (s, 3H) |
| 3.005 | 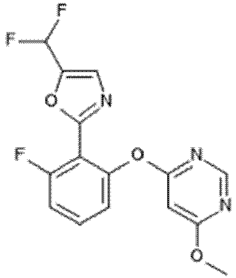 | 8.35 (s, 1H), 7.55 (dt, 1H), 7.40 (t, 1H), 7.22-7.14 (m, 1H), 7.11 (d, 1H), 6.79-6.52 (t, 1H), 6.26 (d, 1H), 3.98 (s, 3H) |
| 3.006 | 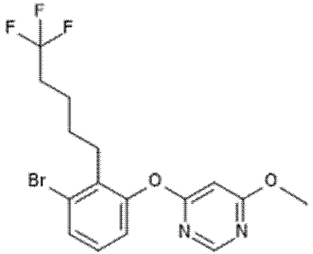 | 8.45 (s, 1H), 7.48 (dd, 1H), 7.13 (t, 1H), 7.01 (dd, 1H), 6.16 (d, 1H), 3.99 (s, 3H), 2.71 (br t, 2H), 2.13-1.99 (m, 2H), 1.62-1.57 (m, 4H) |
| 3.007 | 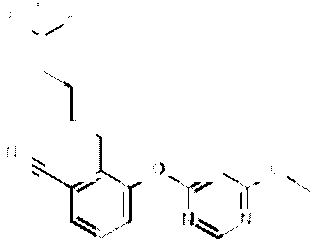 | 8.42 (s, 1H), 7.56 (dd, 1H), 7.40-7.34 (m, 1H), 7.32-7.27 (m, 1H), 6.26 (d, 1H), 4.01 (s, 3H), 2.85-2.77 (m, 2H), 2.13-2.00 (m, 2H), 1.73-1.58 (m, 4H) |

### Biological Examples

Seeds of a variety of test species are sown in standard soil in pots *Amaranthus retoflexus* (AMARE), *Echinochloa crus-galli* (ECHCG), *Setaria faberi* (SETFA)). After cultivation for one day (pre-emergence) or after 8 days cultivation (post-emergence) under controlled conditions in a glasshouse (at 24/16°C, day/night; 14 hours light; 65 % humidity), the plants are sprayed with an aqueous spray solution derived from the formulation of the technical active ingredient in acetone / water (50:50) solution containing 0.5% Tween 20 (polyoxyethelyene sorbitan monolaurate, CAS RN 9005-64-5). Compounds are applied at 250 g/ha unless otherwise stated. The test plants are then grown in a glasshouse under controlled conditions in a glasshouse (at 24/16°C, day/night; 14 hours light; 65 % humidity) and watered twice daily. After 13 days for pre- and post-emergence, the test is evaluated for the percentage damage caused to the plant. The biological activities are shown in the following table on a five-point scale (5 = 81-100%; 4 = 61-80%; 3=41-60%; 2=21-40%; 1=0-20%).

**TABLE B1 Post-emergence Test**

| Compound | AMARE | ECHCG | SETFA |
|---|---|---|---|
| 2.002 | 2 | 1 | 1 |
| 2.003 | 3 | 4 | 4 |
| 2.004 | 4 | 4 | 4 |
| 2.005 | 2 | 2 | 2 |
| 2.006 | 1 | 3 | 3 |
| 2.007 | 2 | 4 | 4 |
| 2.008 | 3 | 1 | 1 |
| 2.017 | 4 | 4 | 4 |

**TABLE B2 Pre-emergence Test**

| Compound | AMARE | ECHCG | SETFA |
|---|---|---|---|
| 2.002 | 2 | 1 | 1 |
| 2.003 | 5 | 5 | 5 |
| 2.004 | 5 | 5 | 5 |
| 2.005 | 2 | 2 | 2 |
| 2.006 | 2 | 5 | 4 |
| 2.007 | 4 | 5 | 5 |
| 2.008 | 4 | 4 | 4 |
| 2.017 | 5 | 5 | 5 |

**TABLE B3 - Comparative Test (Post-Emergence)**

| **Compound** | **RATE g/ha** | **% Phytotoxicity** | |
|---|---|---|---|
| | | **AMARE** | **ZEAMX** |
| 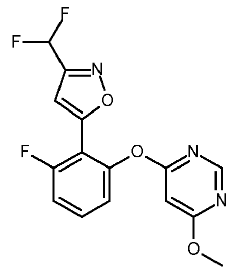 | 250 | 80 | 10 |
| | 63 | 60 | 10 |
| Compound 2.017 | 16 | 60 | 0 |
| 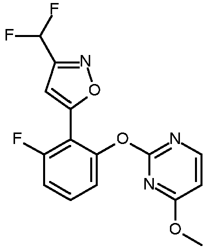 | 250 | 20 | 20 |
| | 63 | 10 | 10 |
| Compound of the type disclosed in WO94/17059 | 16 | 0 | 0 |
| 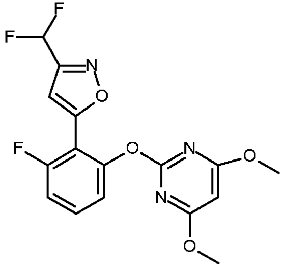 | 250 | 0 | 0 |
| | 33 | 0 | 0 |
| Compound of the type disclosed in WO94/17059 | 16 | 0 | 0 |

The results demonstrate that the 2,4-pyrimidine compounds of the present invention exhibit much improved herbicidal action vis-à-vis weed species e.g AMARE whilst conferring little if any damage to the crop (ZEAMX - corn).

## Claims

1. A compound of Formula (I): 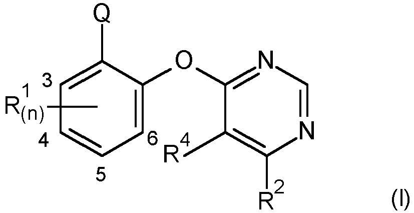
or an agronomically acceptable salt thereof,
wherein
Q is a 5-membered aromatic heterocyclic ring selected from the group consisting of: 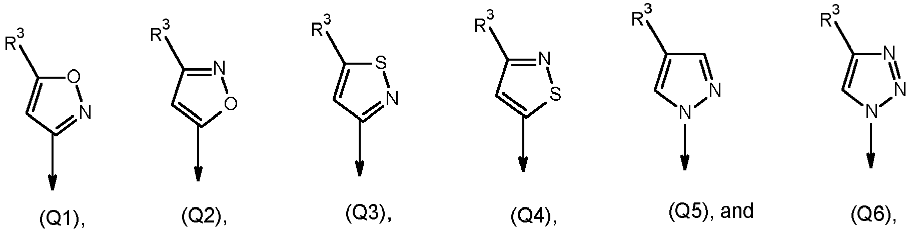
each R¹ is independently selected from the group consisting of halogen, -CN, nitro, C₁-C₄alkyl, C₂-C₄alkenyl, C₂-C₄alkynyl, C₁-C₄haloalkyl, C₁-C₄alkoxy-, C₁-C₄haloalkoxy- and -S(O)ₚC₁-C₄alkyl;
R² is selected from the group consisting of hydrogen, halogen, C₁-C₄alkyl, -NR⁵R⁶, C₁-C₄haloalkyl and C₁-C₄alkoxy;
R³ is selected from the group consisting of hydrogen, C₁-C₄alkyl, C₂-C₄alkenyl, C₂-C₄alkynyl, cyclopropyl, C₁-C₂haloalkyl, C₁-C₂alkoxy-, C₁-C₂haloalkoxy-, halogen, - C(O)C₁-C₄alkyl, NO₂, -CH₂CN, -CN and -S(O)ₚC₁-C₄alkyl;
R⁴ is selected from the group consisting of hydrogen, halogen, -OCF₃, -OCF₂H and - CN;
R⁵ is hydrogen or C₁-C₄alkyl;
R⁶ is hydrogen or C₁-C₄alkyl;
n = 0, 1 or 2; and
p = 0, 1 or 2.

2. A compound of Formula (I) according to claim 1, wherein Q is selected from the group consisting of Q1, Q2 and Q5.

3. A compound of Formula (I) according to claim 1, wherein Q is Q2.

4. A compound according to any one of the previous claims, wherein n is 1 and R¹ is fluorine.

5. A compound according to claim 4, wherein R¹ is 3-fluoro.

6. A compound according to any one of the previous claims, wherein R² is methyl or methoxy.

7. A compound according to any one of previous claims, wherein R⁴ is selected from the group consisting of hydrogen, fluorine and chlorine.

8. A herbicidal composition comprising a compound according to any one of the previous claims and an agriculturally acceptable formulation adjuvant.

9. A herbicidal composition according to claim 8, further comprising at least one additional pesticide.

10. A herbicidal composition according to claim 9, wherein the additional pesticide is a herbicide or herbicide safener.

11. A method of controlling weeds at a locus comprising application to the locus of a weed controlling amount of a composition according to any one of claims 8 to 10.

12. Use of a compound of Formula (I) as defined in claim 1 as a herbicide.

## Patentansprüche

1. Verbindung der Formel (I): 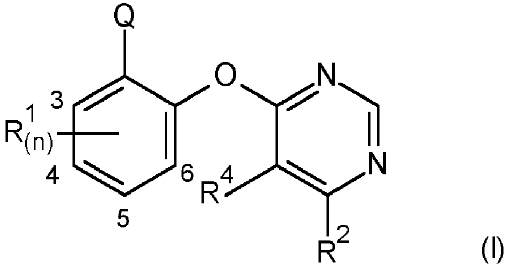
oder ein agronomisch annehmbares Salz davon,
wobei
Q ein 5-gliedriger aromatischer heterocyclischer Ring ist, der ausgewählt ist aus der Gruppe bestehend aus: 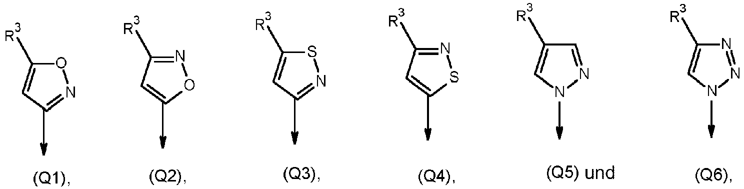
jedes R¹ unabhängig ausgewählt ist aus der Gruppe bestehend aus Halogen, -CN, Nitro, C₁-C₄-Alkyl, C₂-C₄-Alkenyl, C₂-C₄-Alkinyl, C₁-C₄-Halogenalkyl, C₁-C₄-Alkoxy-, C₁-C₄-Halogenalkoxy- und -S(O)ₚC₁-C₄-Alkyl;
R² ausgewählt ist aus der Gruppe bestehend aus Wasserstoff, Halogen, C₁-C₄-Alkyl, -NR⁵R⁶, C₁-C₄-Halogenalkyl und C₁-C₄-Alkoxy;
R³ ausgewählt ist aus der Gruppe bestehend aus Wasserstoff, C₁-C₄-Alkyl, C₂-C₄-Alkenyl, C₂-C₄-Alkinyl, Cyclopropyl, C₁-C₂-Halogenalkyl, C₁-C₂-Alkoxy-, C₁-C₂-Halogenalkoxy-, Halogen, -C (O) C₁-C₄-Alkyl, NO₂, -CH₂CN, - CN und -S(O)ₚC₁-C₄-Alkyl;
R⁴ ausgewählt ist aus der Gruppe bestehend aus Wasserstoff, Halogen, -OCF₃, -OCF₂H und -CN;
R⁵ Wasserstoff oder C₁-C₄-Alkyl ist;
R⁶ Wasserstoff oder C₁-C₄-Alkyl ist;
n = 0, 1 oder 2; und
p = 0, 1 oder 2.

2. Verbindung der Formel (I) nach Anspruch 1, wobei Q ausgewählt ist aus der Gruppe bestehend aus Q1, Q2 und Q5.

3. Verbindung der Formel (I) nach Anspruch 1, wobei Q Q2 ist.

4. Verbindung nach einem der vorstehenden Ansprüche, wobei n 1 ist und R¹ Fluor ist.

5. Verbindung nach Anspruch 4, wobei R¹ 3-Fluor ist.

6. Verbindung nach einem der vorstehenden Ansprüche, wobei R² Methyl oder Methoxy ist.

7. Verbindung nach einem der vorstehenden Ansprüche, wobei R⁴ ausgewählt ist aus der Gruppe bestehend aus Wasserstoff, Fluor und Chlor.

8. Herbizide Zusammensetzung, umfassend eine Verbindung nach einem der vorstehenden Ansprüche und ein landwirtschaftlich annehmbares Formulierungsadjuvans.

9. Herbizide Zusammensetzung nach Anspruch 8, ferner umfassend mindestens ein zusätzliches Pestizid.

10. Herbizide Zusammensetzung nach Anspruch 9, wobei das zusätzliche Pestizid ein Herbizid oder ein Herbizid-Safener ist.

11. Verfahren zum Bekämpfen von Unkraut an einem Locus, umfassend das Ausbringen einer unkrautbekämpfenden Menge einer Zusammensetzung nach einem der Ansprüche 8 bis 10 auf den Locus.

12. Verwendung einer Verbindung der Formel (I), wie in Anspruch 1 definiert, als Herbizid.

## Revendications

1. Composé de formule (I) : 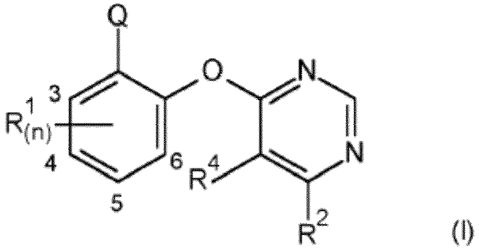
ou sel acceptable sur le plan agronomique correspondant,
Q étant un cycle hétérocyclique aromatique à 5 chaînons choisi dans le groupe constitué par : 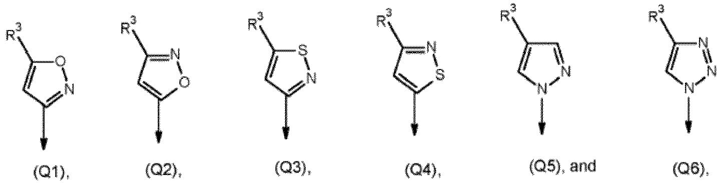
chaque R¹ étant indépendamment choisi dans le groupe constitué par halogène, -CN, nitro, alkyle en C₁₋₄, alcényle en C₂₋₄, alcynyle en C₂₋₄, halogénoalkyle en C₁₋₄, alcoxy en C₁₋₄, halogénoalcoxy en C₁₋₄ et -S(O)ₚ-alkyle en C₁₋₄ ;
R² étant choisi dans le groupe constitué par hydrogène, halogène, alkyle en C₁₋₄, -NR⁵R⁶, halogénoalkyle en C₁₋₄ et alcoxy en C₁₋₄ ;
R³ étant choisi dans le groupe constitué par hydrogène, alkyle en C₁₋₄, alcényle en C₂₋₄, alcynyle en C₂₋₄, cyclopropyle, halogénoalkyle en C₁₋₂, alcoxy en C₁₋₂, halogénoalcoxy en C₁₋₂, halogène, -C(O)-alkyle en C₁₋₄, NO₂, -CH₂CN, -CN et -S(O)ₚ-alkyle en C1-4 ;
R⁴ étant choisi dans le groupe constitué par hydrogène, halogène, -OCF₃, -OCF₂H et -CN ;
R⁵ étant hydrogène ou alkyle en C₁₋₄ ;
R⁶ étant hydrogène ou alkyle en C₁₋₄ ;
n = 0, 1 ou 2 ; et
p = 0, 1 ou 2.

2. Composé de formule (I) selon la revendication 1, Q étant choisi dans le groupe constitué par Q1, Q2 et Q5.

3. Composé de formule (I) selon la revendication 1, Q étant Q2.

4. Composé selon l'une quelconque des revendications précédentes, n étant 1 et R¹ étant fluor.

5. Composé selon la revendication 4, R¹ étant 3-fluoro.

6. Composé selon l'une quelconque des revendications précédentes, R² étant méthyle ou méthoxy.

7. Composé selon l'une quelconque des revendications précédentes, R⁴ étant choisi dans le groupe constitué par hydrogène, fluor et chlore.

8. Composition herbicide comprenant un composé selon l'une quelconque des revendications précédentes et un adjuvant de formulation acceptable sur le plan agricole.

9. Composition herbicide selon la revendication 8, comprenant en outre au moins un pesticide supplémentaire.

10. Composition herbicide selon la revendication 9, le pesticide supplémentaire étant un herbicide ou un phytoprotecteur herbicide.

11. Procédé de régulation de mauvaises herbes au niveau d'un site comprenant une application sur le site d'une quantité de régulation de mauvaises herbes d'une composition selon l'une quelconque des revendications 8 à 10.

12. Utilisation d'un composé de formule (I) tel que défini dans la revendication 1 en tant qu'herbicide.
